# EUROPEAN PATENT APPLICATION

(11) **EP 4 421 165 A1**
(43) Date of publication of application: **28.08.2024**
(21) Application number: 22960558.9
(22) Date of filing: 08.11.2022
(51) Int. Cl.: C12N 9/12, C12N 15/54, C12N 15/113, C12N 15/84, A01H 5/04, A01H 5/12, A01H 5/10, A01H 1/02, A01H 6/20

(54) **RAPE GREEN REVOLUTION GENE BGR AND USE THEREOF**

(30) Priority: 26.09.2022 CN 202211177399
(71) Applicant: Institute of Genetics and Developmental Biology Chinese Academy of Sciences, Beijing 100101 (CN)
(72) Inventor: HU, Zanmin, Beijing 100101 (CN); FAN, Chengming, Beijing 100101 (CN); GUO, Xupeng, Beijing 100101 (CN); LI, Shuangshuang, Beijing 100101 (CN); CHEN, Yuhong, Beijing 100101 (CN)
(74) Representative: Hughes, Sean David
(86) International application number: PCT/CN2022/130530
(87) International publication number: WO 2024/065959

(57) **Abstract**

Provided are a rape green revolution gene bgr and a use thereof. The gene bgr can significantly reduce the height of rape plants, achieve thick and strong stems, lodging resistance, a reduced branching angle, and a compact plant type, and can effectively increase the harvest index. The gene has remarkable heterosis, heterozygous bgr can enable tetraploid rape (*Brassica napus* and *Brassica juncea*) to have more ideal plant type characteristics, the plant height is 120-160 cm, the plant type is compact, the branching angle is small (less than 30°), the first branching position is lowered, the harvest index is increased by about 29.03%, the gene is suitable for close planting, and the yield is greatly increased by 11-27%. The oil content of hybrid Brassica napus having bgr is greatly increased and can reach 51.7-54.86%, and compared with the oil contents of common high-oil-content rape varieties, the oil content is increased by 9.14-19.26%. A valuable genetic resource is provided for rape breeding, and an effective means is provided for creation of new rape germplasm.

## Description

### Cross-reference to related application

The present application claims priority to Chinese Patent Application No. 202211177399.1, entitled "Oilseed rape Green Revolution Gene bgr and Use Thereof' filed on September 26, 2022, the entire disclosure of which is incorporated herein by reference in its entirety.

### Technical Field

The present application relates to the technical field of plant genetic engineering, in particular to an oilseed rape green revolution gene *bgr* and use thereof.

### Background Art

*Brassica napus* (oilseed rape) is an important oilseed crop. Oilseed rape belongs to the family of Brassicaceae, genus of *Brassica.* In the 1930s, Japanese scholars Sheng Yonghe, Nagaharu U and others divided *Brassica* plants into three diploid species *Brassica rapa* (AA, 2n=20), *Brassica nigra* (BB, 2n=16) and *Brassica oleracea* (CC, 2n = 16), and three allotetraploid species *Brassica napus* (AACC, 2n=38), *Brassica juncea* (AABB, 2n=36) and *Brassica carinata* (BBCC, 2n=34), which were spontaneously hybridized in a pairwise manner. At present, the main cultivars of oilseed rape in China are mainly *B. napus,* and *B. juncea* and *B. rapa* are also planted in small areas. The oilseed rape planting area in China is about 100 million mu (i.e., 6.67 million ha). Europe, Canada and India are also important production areas for oilseed rapeseed.

Looking at the present situation of oilseed rapeseed breeding at home and abroad, although oilseed rapeseed breeding has realized the improvement of quality such as "low erucic acid and low glucosinolate" and oil content, the overall average yield per unit area of oilseed rape is low.

In the last century, the successful application of "Green Revolution" gene in wheat, rice and other crops has improved its plant architecture, such as semi-dwarfing, which in turn has enhanced its lodging resistance and greatly improved its yield and harvest index. In view of the common problems of existing oilseed rape varieties, such as high plant height, wide canopy and easy lodging, plant architecture breeding is the focus of oilseed rape breeding. The ideal plant architecture of oilseed rape refers to a semi-dwarfing plant architecture with a plant height of 120-140 cm and a compact plant architecture having a branching angle of <30°, in which the siliques are erect, dense and medium in length. The "Green Revolution" gene has not been applied in oilseed rape breeding, so the search for the "Green Revolution" gene in oilseed rape to obtain the new germplasm with the ideal plant architecture characteristics is the key to break the bottleneck of oilseed rape plant architecture breeding in the world.

### References:

1. Nagaharu U: Genomic analysis in Brassica with special reference to the experimental formation of B. napus and peculiar mode of fertilization. Japanese Journal of Botany 1935:389-452.
2. Fu T, Zhou Y: Progress and future development of hybrid oilseed rapeseed in China. Engineering Sciences 2013, 11(05):11-13.
3. Deblock M, Debrouwer D, Tenning P: Transformation of Brassica napus and Brassica oleracea using Agrobacterium tumefaciens and the expression of the Bar and Neo genes in the transgenic plants. Plant Physiology 1989, 91(2):694-701.
4. Xin P, Yan J, Fan J, Chu J, Yan C: An improved simplified high-sensitivity quantification method for determining brassinosteroids in different tissues of rice and Arabidopsis. Plant Physiology 2013, 162(4):2056-2066.
5. Emms DM, Kelly S: OrthoFinder: Phylogenetic orthology inference for comparative genomics. Genome Biology 2019, 20(1):238.
6. Jiang YY, Chai YP, Lu MH, Han XL, Lin Q, Zhang Y, Zhang Q, Zhou Y, Wang XC, Gao C et al: Prime editing efficiently generates W542L and S621I double mutations in two ALS genes in maize. Genome Biology 2020, 21(1):257.

### Summary

The purpose of the present application is to provide an oilseed rape green revolution gene *bgr* and use thereof.

Another purpose of the present application is to provide a method for creating new germplasm of *Brassica napus.*

In order to achieve the purposes of the present application, in a first aspect, the present application provides an oilseed rape green revolution gene *bgr,* which is a gene encoding the following protein (a) or (b):
(a1) a protein comprising the amino acid sequence represented by SEQ ID NO:4;
(b1) a protein derived from (a1) by substitution, deletion or addition of one or several amino acids in the sequence represented by SEQ ID NO:4 and having an equivalent function.

The genomic DNA sequence of the gene *bgr* is as follows:
i) the nucleotide sequence represented by SEQ ID NO:1;
ii) the nucleotide sequence obtained by substitution, deletion and/or addition of one or more nucleotides in the sequence represented by SEQ ID NO:1 and expressing an equivalent functional protein;
iii) the nucleotide sequence that hybridizes to the sequence represented by SEQ ID NO:1 under stringent conditions and expressing an equivalent functional protein, wherein the stringent conditions are hybridization in a solution of 0.1 × SSPE containing 0.1% SDS or a solution of 0.1×SSC containing 0.1% SDS at 65°C and washing the membrane with the solution; or
iv) the nucleotide sequence having 90% or more homology with the nucleotide sequence of i), ii) or iii) and expressing an equivalent functional protein.

The CDS sequence of gene *bgr* is as follows:
i) the nucleotide sequence represented by SEQ ID NO:3;
ii) the nucleotide sequence obtained by substitution, deletion and/or addition of one or more nucleotides in the sequence represented by SEQ ID NO:3 and expressing an equivalent functional protein;
iii) the nucleotide sequence that hybridizes to the sequence represented by SEQ ID NO:3 under stringent conditions and expressing an equivalent functional protein, wherein the stringent conditions are hybridization in a solution of 0.1 × SSPE containing 0.1% SDS or a solution of 0.1×SSC containing 0.1% SDS at 65°C and washing the membrane with the solution; or
iv) the nucleotide sequence having 90% or more homology with the nucleotide sequence of i), ii) or iii) and expressing an equivalent functional protein.

The oilseed rape green revolution gene *bgr* is derived from a mutant of *Brassica rapa, bgr-JT. bgr-JT* is a plant-dwarfing, compact branching mutant of *Brassica rapa* obtained by chemical mutagenesis of *Brassica rapa* Jietou No.2 (diploid, 2n = 20, genome AA) with ethyl methanesulfonate (EMS), which has the characteristics of ideal plant architecture.

In a second aspect, the present application provides homologous genes of the oilseed rape green revolution gene *bgr,* totaling seven homologous genes, which are mBnaA01T000997, mBnaA03T004751, mBnaA08T002216, mBnaC03T001151, mBnaC04T001741, mBnaC04T005325 and mBnaC07T004394, respectively;
wherein, mBnaA01T000997 is a gene encoding the following protein (a2) or (b2):
   (a2) a protein comprising the amino acid sequence represented by SEQ ID NO:23;
   (b2) a protein derived from (a2) by substitution, deletion or addition of one or several amino acids in the sequence represented by SEQ ID NO:23 and having an equivalent function;
mBnaA03T004751 is a gene encoding the following protein (a3) or (b3):
   (a3) a protein comprising the amino acid sequence represented by SEQ ID NO:25;
   (b3) a protein derived from (a3) by substitution, deletion or addition of one or several amino acids in the sequence represented by SEQ ID NO:25 and having an equivalent function;
mBnaA08T002216 is a gene encoding the following protein (a4) or (b4):
   (a4) a protein comprising the amino acid sequence represented by SEQ ID NO:27;
   (b4) a protein derived from (a4) by substitution, deletion or addition of one or several amino acids in the sequence represented by SEQ ID NO:27 and having an equivalent function;
mBnaC03T001151 is a gene encoding the following protein (a5) or (b5):
   (a5) a protein comprising the amino acid sequence represented by SEQ ID NO:29;
   (b5) a protein derived from (a5) by substitution, deletion or addition of one or several amino acids in the sequence represented by SEQ ID NO:29 and having an equivalent function;
mBnaC04T001741 is a gene encoding the following protein (a6) or (b6):
   (a6) a protein comprising the amino acid sequence represented by SEQ ID NO:31;
   (b6) a protein derived from (a6) by substitution, deletion or addition of one or several amino acids in the sequence represented by SEQ ID NO:31 and having an equivalent function;
mBnaC04T005325 is a gene encoding the following protein (a7) or (b7):
   (a7) a protein comprising the amino acid sequence represented by SEQ ID NO:33;
   (b7) a protein derived from (a7) by substitution, deletion or addition of one or several amino acids in the sequence represented by SEQ ID NO:33 and having an equivalent function;
mBnaC07T004394 is a gene encoding the following protein (a8) or (b8):
   (a8) a protein comprising the amino acid sequence represented by SEQ ID NO:35;
   (b8) a protein derived from (a8) by substitution, deletion or addition of one or several amino acids in the sequence represented by SEQ ID NO: 35 and having an equivalent function.

In a third aspect, the present application provides a biomaterial containing the oilseed rape green revolution gene *bgr* or the homologous gene, wherein the biomaterial includes but is not limited to a recombinant DNA, an expression cassette, a transposon, a plasmid vector, a virus vector or an engineered bacterium.

In a fourth aspect, the present application provides any of the following uses of the oilseed rape green revolution gene *bgr* or the homologous gene or the biomaterial:
(1) for regulating plant height, plant architecture, leaf type, oil content and yield;
(2) for improving plant variety; and
(3) for preparing transgenic plants.

The plant architecture includes but is not limited to branching angle and branch number.

In the present application, the plants are *Brassica* plants, preferably oilseed rape, more preferably tetraploid oilseed rape, and most preferably *Brassica napus* and *Brassica juncea.*

In a fifth aspect, the present application provides a method for reducing plant height, reducing branching angle, compacting plant architecture, shrinking leaves, increasing oil content, and increasing yield of oilseed rape, which comprises the following steps: introducing mutation into oilseed rape genome by means of genetic engineering, gene editing, chemical and/or physical mutagenesis and plant hybridization, so that the E in the conserved motif LGTPTRE in the encoded glycogen synthase kinase 3β (GSK3β) and its homologous protein is mutated into K; or,

introducing the oilseed rape green revolution gene *bgr* or the homologous gene into oilseed rape via plasmids or integrating the oilseed rape green revolution gene *bgr* or the homologous gene into oilseed rape chromosome by genetic engineering.

Preferably, the oilseed rape is a tetraploid, more preferably *Brassica napus* and *Brassica juncea.*

Further, the method includes overexpressing the oilseed rape green revolution gene *bgr* or the homologous gene in plants;

the overexpression mode is selected from the following modes 1) to 5), or any combination thereof:
1) by introducing a plasmid having the gene;
2) by increasing the copy number of the gene on the plant chromosome;
3) by changing the promoter sequence of the gene on the plant chromosome;
4) by operably linking a strong promoter or a weak promoter with the gene; and
5) by introducing an enhancer.

Preferably, the promoter is the promotor of *bgr* or *BGR* gene encoding GSK3β protein in *Brassica rapa,* and a nucleotide sequence of the promoter is:
i) the nucleotide sequence represented by SEQ ID NO:7;
ii) the nucleotide sequence obtained by substitution, deletion and/or addition of one or more nucleotides in the sequence represented by SEQ ID NO:7 and having an equivalent function;
iii) a nucleotide sequence that hybridizes to the sequence represented by SEQ ID NO:7 under stringent conditions, wherein the stringent conditions are hybridization in a solution of 0.1×SSPE containing 0.1% SDS or a solution of 0.1×SSC containing 0.1% SDS at 65°C and washing the membrane with the solution; or
iv) a nucleotide sequence having 90% or more homology with the nucleotide sequence of i), ii) or iii) and having the same promoter function.

In a sixth aspect, the present application provides the use of transgenic oilseed rape obtained by the method in plant breeding.

The breeding method includes, but is not limited to, transgenesis, hybridization, backcrossing, self-crossing or asexual reproduction.

Preferably, the obtained transgenic oilseed rape is used as a maintainer line to create a sterile line (for example *bgr*A) or a restorer line (for example PM112) of oilseed rape.

In a seventh aspect, the present application provides a method for creating a new germplasm of *Brassica napus,* in which a mutation is introduced into the genome of diploid oilseed rape by using genetic engineering technology such as gene edition or mutagenesis, so that the E in the conserved motif LGTPTRE in the encoded glycogen synthase kinase 3β is mutated into K (as shown in SEQ ID NO:4), and an oilseed rape mutant with ideal plant architecture characteristics is obtained; then the oilseed rape mutant is hybridized with *Brassica napus* using microspore culture technology to obtain *Brassica napus* breeding materials.

In a specific embodiment of the present application, CRISPR/Cas9 gene editing technology is used to introduce mutations into the diploid oilseed rape genome, and the prime editing sequence is preferably represented by SEQ ID NO:36.

In an eighth aspect, the present application provides a method for creating a new germplasm of *Brassica napus,* in which a mutation is introduced into the genome of tetraploid oilseed rape by using genetic engineering technology such as gene edition or mutagenesis, so that the E in conserved motif LGTPTRE in the encoded glycogen synthase kinase 3β is mutated into K (as shown in SEQ ID NO:4), and an oilseed rape mutant with ideal plant architecture characteristics is obtained.

In a ninth aspect, the present application provides a glycogen synthase kinase 3β mutant comprising a mutation of E in the conserved motif LGTPTRE in glycogen synthase kinase 3β from E to an amino acid other than E (preferably a mutation from E to K). The glycogen synthase kinase 3β is derived from oilseed rape.

In a tenth aspect, the present application provides a method for breeding a new variety of *Brassica napus,* in which a sterile line or restorer line of dwarf oilseed rape is created on the basis of obtaining oilseed rape mutants with ideal plant characteristics using the above-described genetic engineering technology such as (gene edition or transgenesis) or mutagenesis, and a hybrid is produced with three-line matching.

The present application has at least the following advantages and beneficial effects by means of the above technical solutions:
The present application has discovered for the first time the oilseed rape green revolution gene *bgr* (SEQ ID NO:1 and SEQ ID NO:3), which can significantly reduce the height of oilseed rape plants, make the stems thick and strong, realize lodging resistance, reduce the branching angle, compact the plant architecture and effectively improve the harvest index. The gene has significant heterosis, and the heterozygous *bgr* can enable tetraploid oilseed rape (*Brassica napus* and *Brassica juncea*) to have more ideal plant architecture, wherein the plant height is 120 to 160 cm, the plant architecture is compact, and the branching angle is small (<30°, the existing branching angle of oilseed rape is greater than 30°), the position of the first branching is lowered, the harvest index is improved by about 29.03%, the method is suitable for dense planting (30,000 to 40,000 plants/mu (i.e., 450,000 to 600,000 plants/ha), while the current planting density of *Brassica napus* is 15,000 to 20,000 plants/mu (i.e., 225,000 to 300,000 plants/ha)), the yield is significantly increased by 11% to 27%. The oil content of hybrid *Brassica napus* with *bgr* is substantially increased up to 51.7% to 54.86%, which is 9.14% to 19.26% higher than that of common high-oil oilseed rape varieties (46.00% to 47.37%). The present application contributes valuable gene resources for oilseed rape breeding, and provides an effective means for the creation of new oilseed rapeseed germplasm.

### Brief Description of the Drawings

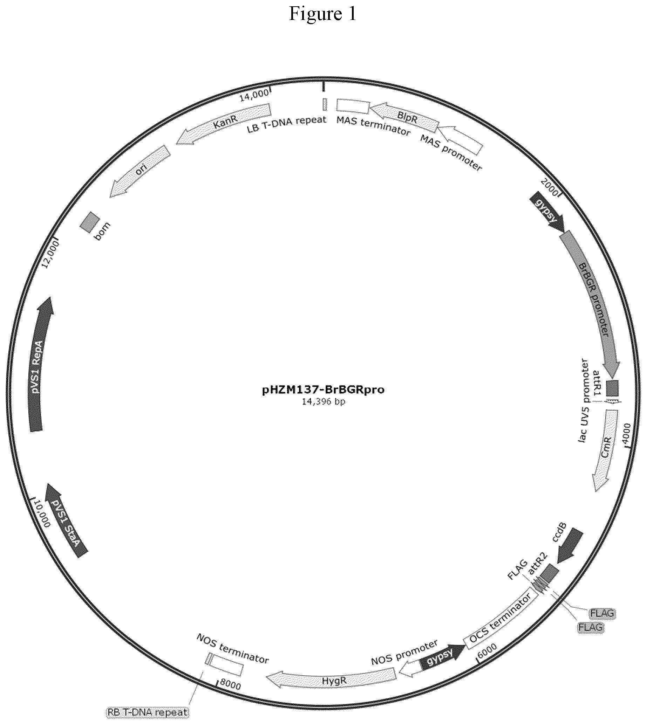

Figure 1 is a diagram of pHZM137-BrBGRpro vector in a preferred example of the present application.

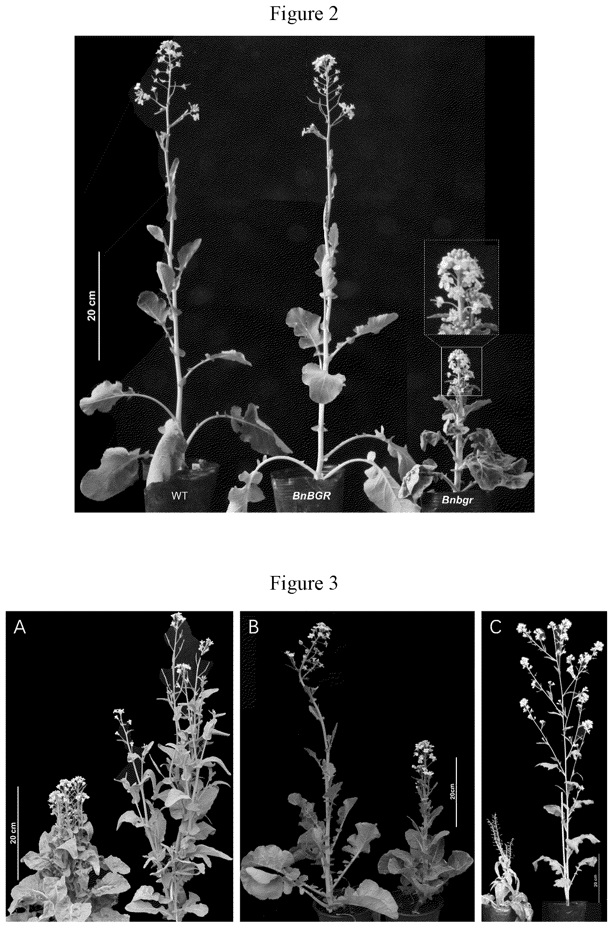

Figure 2 shows the functional analysis of *BrBGR* and *Brbgr* in *Brassica napus* in a preferred example of the present application. Wherein, WT denotes wild type Westar; *BGR* denotes a phenotype of *BrBGR* gene transgenic strain driven by its own promoter; *bgr* denotes the phenotype of *Brbgr* gene transgenic strain driven by its own promoter.
Figure 3 shows the effect of *bgr* on the plant architectures of different *Brassica* plants in a preferred example of the present application. Wherein, A shows hybrid phenotype of *bgr-JT,* a mutant *Brassica rapa* Jietou No.2, and *Brassica rapa* Yellow Sarson, with F1 on the left and Yellow Sarson on the right; B shows hybrid phenotype of *bgr-JT,* a mutant *Brassica rapa* Jietou No.2, and *Brassica napus* Westar, with Westar on the left and F1 on the right; C shows the hybrid phenotype of *bgr-JT,* a mutant *Brassica rapa* Jietou No.2, and *Brassica juncea* FY02, with FY02 on the right and F1 on the left.

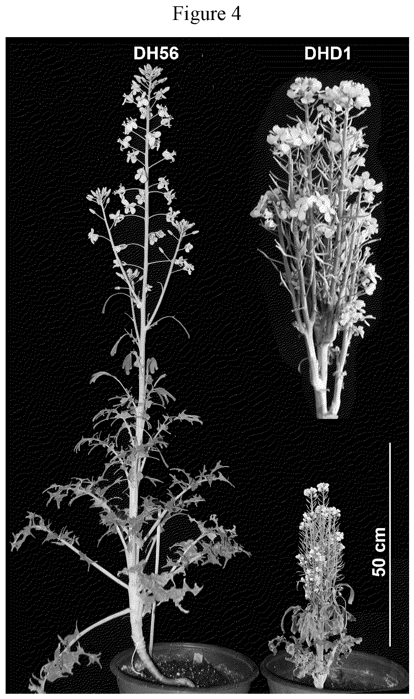

Figure 4 shows some of the DH strains of *Brassica napus* in a preferred example of the present application. Wherein, DH56 and DHD1 are tall and dwarf lines of *Brassica napus* isolated from microspore culture, respectively.

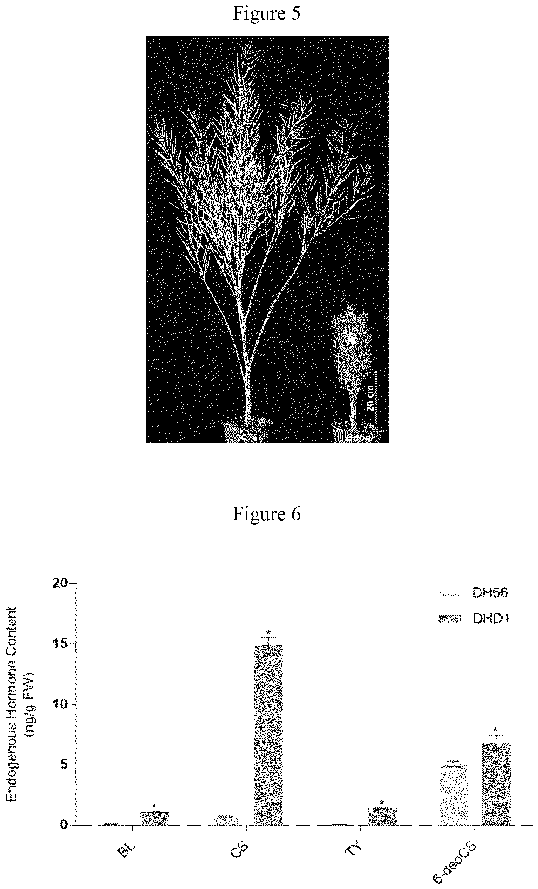

Figure 5 shows a *Brassica napus* mutant *bgr* in a preferred example of the present application. Wherein, C76 denotes *Brassica napus; Brassica napus bgr* strain DHD1.
Figure 6 shows the determination of metabolites in the brassinolide synthesis pathway of *Brassica napus Bnbgr* strain DHD1 in a preferred example of the present application. Wherein, D1 denotes dwarf *Bnbgr* strain DHD1; DH56 denotes a high-stem *Brassica napus* DH56. BL denotes brassinolide, CS denotes castasterone, TE denotes teasterone, TY denotes typhasterol, and 6-deoCS denotes 6-deoxocastasterone. FW denotes the fresh weight of tissue. Wherein, * means P<0.05.

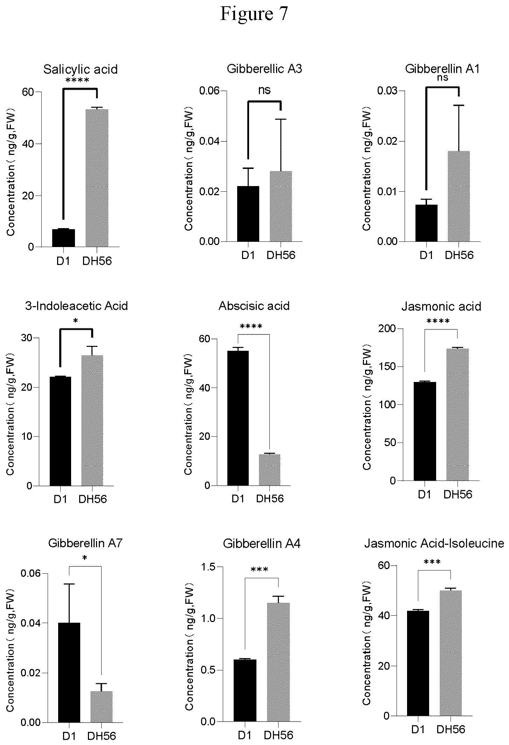

Figure 7 shows the determination of different hormones in a preferred example of the present application. Wherein, D1 denotes dwarf *Bnbgr* strain DHD1; DH56 denotes a high-stem *Brassica napus* DH56. Salicylic acid; Gibberellin A3; Gibberellin A1; Indole-3-acetic acid; Abscisic acid; Jasmonic acid; Gibberellin A7; Gibberellin A4; and Jasmonoyl-isoleucine. FW denotes the fresh weight of tissue. Wherein, * means P<0.05, *** means P<0.001, and **** means p <0.0001.

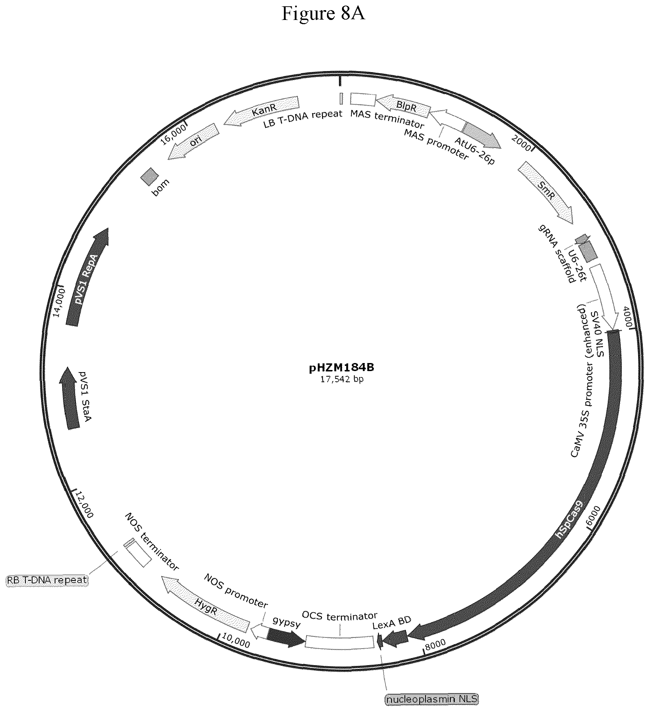

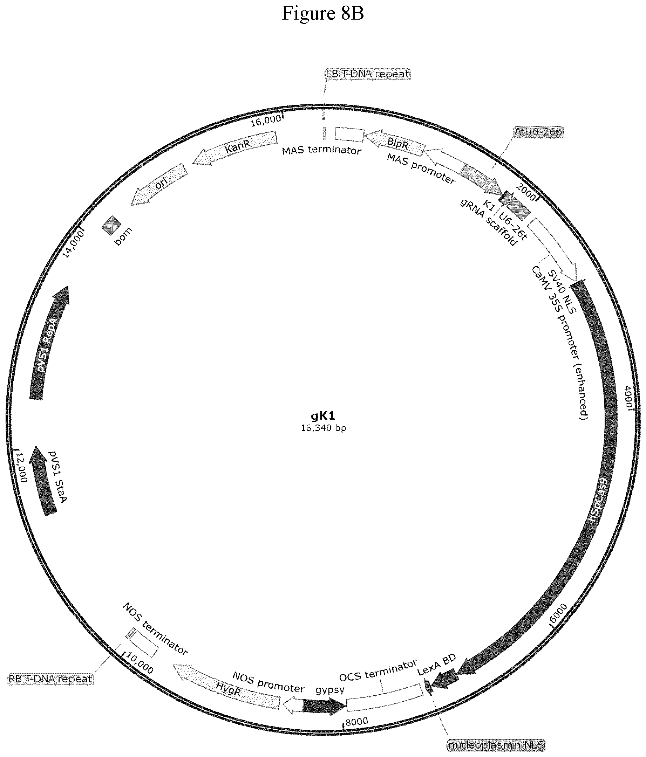

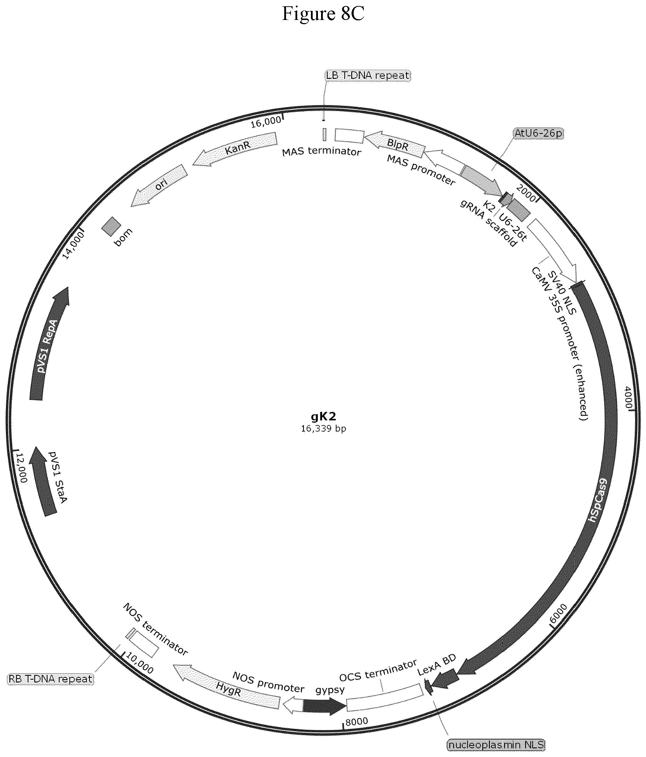

Figures 8A-8C are schematic diagrams of gene-edited vectors in a preferred example of the present application. In Figure 8A, pHZM184B denotes the edited vector constructed in our laboratory; in Figure 8B, gK1 denotes a vector with the K1 locus edited; in Figure 8C, gK2 denotes a vector with the K2 locus edited.

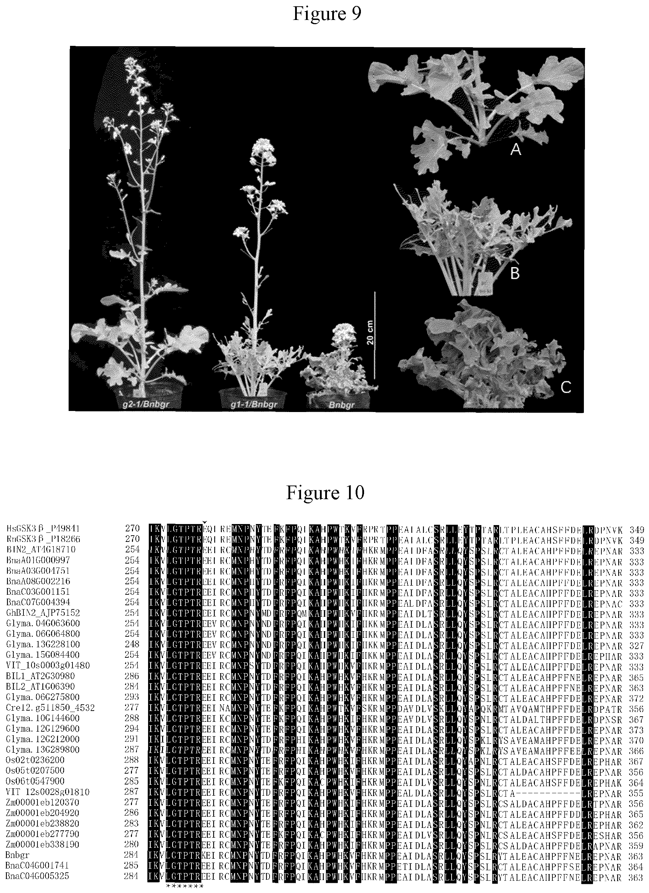

Figure 9 shows the phenotypes of some of the *bgr* knockouts in a preferred example of the present application. Wherein, g2-1/*Bnbgr* denotes a phenotype with the K2 locus edited under the background of *bgr*; g1*-*1/*Bnbgr* denotes a phenotype with the K1 locus edited under the background of *bgr*; and *Bnbgr* denotes dwarf *Brassica napus bgr*; A, B and C are enlarged views of the rosette leaves of g2-1/*Bnbgr,* g1-1/*Bnbgr* and *Bnbgr,* respectively.
Figure 10 is the result of protein sequence alignment in a preferred example of the present application. Wherein, BnaA01T000997, BnaA03T004751, BnaA08T002216, BnaC03T001151, BnaC04T001741, BnaC04T005325 and BnaC07T004394 were derived from a *Brassica napus* mutant DHD1. BIN2, AT4G18710; BII,1, AT2G30980; BII,2, AT1G06390. The amino acid residue indicated by the arrow is the location of BGR mutation (E293K), and * is a conserved motif. HsGSK3β, P49841; Hs, *Homo sapiens*; RnGSK3β, P18266; Rn, *Rattus norvegicu*; the amino acid residue indicated by the arrow is the location of BGR mutation (E293K). Shading indicates that the amino acid residues at this position are exactly the same. Cre12.g511850_4532.3, derived from the genome of *Chlamydomonas reinhardtii.* GhBIN2, AJP75152, BIN2 of cotton. Glyma.13G289800, Glyma.12G212000, Glyma.12G129600, Glyma.10G144600, Glyma.06G275800, Glyma.15G084400, Glyma.13G228100, Glyma.06G064800 and Glyma.04G063600 were derived from the soybean genome. Zm00001eb338190, Zm00001eb277790, Zm00001eb238820, Zm00001eb204920 and Zm00001eb120370 were derived from the maize genome. VIT_12s0028g01810 and VIT_10s0003g01480 were derived from the grape genome. Os06t0547900, Os05t0207500 and Os02t0236200 were derived from the rice genome.

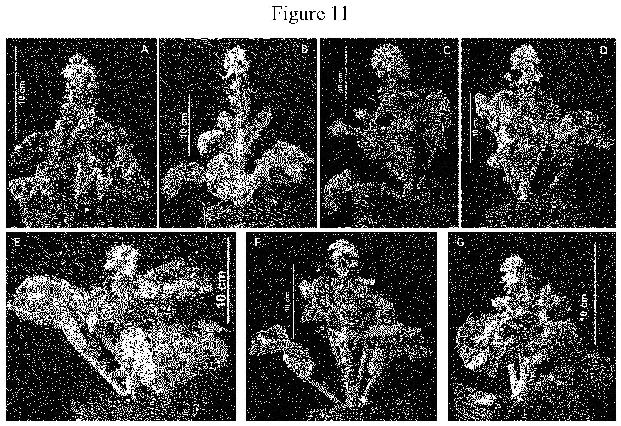

Figure 11 shows the phenotype of the transgenic plant of *bgr* homologous gene mutant in a preferred example of the present application. Wherein, A-G are the phenotypes of transgenic plants of *mBnaA01T000997, mBnaA03T004751, mBnaA08T002216, mBnaC03T001151, mBnaC04T001741, mBnaC04T005325* and *mBnaC07T004394* mutants, respectively.

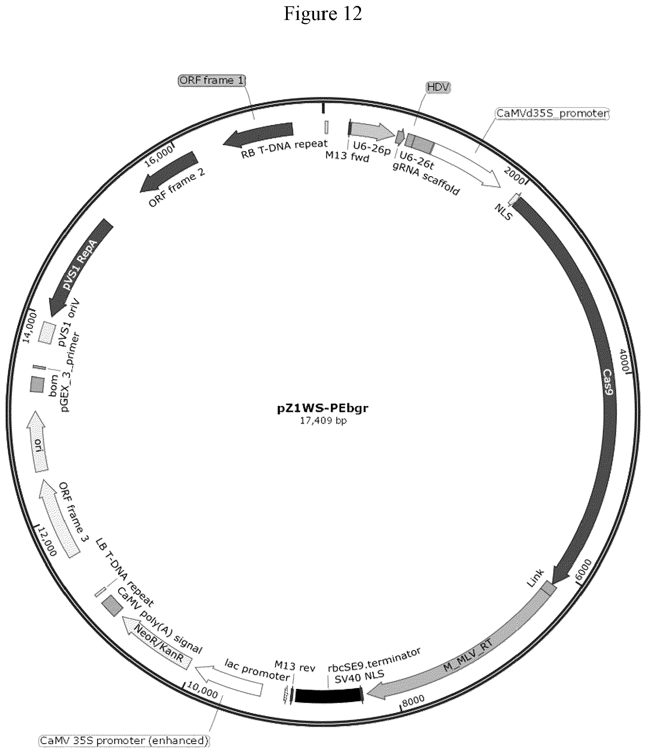

Figure 12 is a diagram showing a vector of BGR prime editing sequence in a preferred example of the present application. Wherein, M_MLV_RT denotes Moloney Murine Leukemia Virus Reverse Transcriptase. The 840H of Cas9 and SpCas9 was mutated to A.

### Specific Modes for Carrying Out the Embodiments

The present application establishes a set of efficient creation systems of new germplasm of *Brassica napus.* In this system, the mutant having breeding potential with ideal plant architecture characteristics was obtained by EMS mutation of diploid oilseed rape. By crossing the mutant with tetraploid oilseed rape in combination with microspore culture technology, the rapid fixation of traits and homozygosity of materials were realized, and finally the *Brassica napus* breeding materials with breeding potential were obtained.

The present application has found for the first time and utilized the ideal plant architecture gene *bgr* of oilseed rape. The ideal plant architecture gene is a gain-of-function gene caused by point mutation, and the E in the conserved sequence LGTPTRE of the encoded protein BGR is mutated into K.

Furthermore, the *bgr* gene is used to improve tetraploid oilseed rape.

Furthermore, the homozygous *bgr* is hybridized with conventional tetraploid oilseed rape to obtain F 1 with heterosis.

Furthermore, the homozygous *bgr Brassica napus* can be used as the maintainer line for the cytoplasmic male sterile line, and the dwarfing and compact plant architecture can be transformed into a sterile line with dwarfing and compact plant architecture through hybridization and continuous backcross.

Furthermore, the above obtained sterile lines with dwarfed and compact plants are hybridized with restorer lines to obtain F 1 seeds, which can be used for oilseed rape production and can be used for dense planting to obtain oilseed rape seeds with high yield and high oil content.

Through breeding analysis, it was found that *bgr* gene had no cross breeding potential in diploid oilseed rape background, but had great breeding potential in tetraploid oilseed rape background.

Furthermore, the *bgr* gene controlling the ideal plant architecture of oilseed rape was obtained by map-based cloning. Through sequence analysis, it was found that the phenotype was obtained by a mutation from G to A at base 2191 in exon 9 of the BGR gene, which results in a transition from E (glutamic acid) to K (lysine) at position 293 in the amino acid sequence encoded by the BGR gene. The gene encodes a glycogen synthase kinase 3β (GSK3β). *bgr* gene is a gain-of-function gene that encodes a protein with a mutation site within the conserved LGTPTRE motif of animal and plant GSK3β, in which E is mutated into K.

Through further homologous gene analysis, it was found that there were 8 copies (8 homologous genes) in oilseed rape genome. By site-directed mutagenesis, 7 other copies were found to be mutated in the same way as *bgr* (Figure 11), which could all cause phenotypes similar to *bgr.* Moreover, the proteins encoded by homologous genes of other species in which E within their conserved GTPTRE motifs is mutated into K all have the function of regulating the oilseed rape plant architecture. It can be seen that the mutation from E to K within the LGTPTRE motif of this kind of protein can all dwarf oilseed rape plants and reduce the branching angle.

Furthermore, transforming E into K within the LGTPTRE of BGR proteins by gene editing can result in dwarfed plants and compact *Brassica napus* plants.

Furthermore, by targeted mutation of the BGR gene (genomic DNA and its cDNA) to *bgr* (genomic DNA and its cDNA) *in vitro* and transfection of the mutated gene *bgr* into tetraploid oilseed rape results in plant dwarfing and compact oilseed rape breeding materials.

Furthermore, the *BGR*/*bgr* intrinsic promoter is utilized to drive the *bgr* gene for tetraploid oilseed rape improvement.

The following examples are used to illustrate the present application, but are not used to limit the scope of the present application. Unless otherwise indicated, the examples are all based on conventional experimental conditions, such as those described in Sambrook J & Russell DW, Molecular Cloning: a Laboratory Manual, 2001, or the conditions suggested by the manufacturer's instructions.

### Example 1: Map based cloning of bgr gene in Brassica rapa

### 1. Mapping of Brbgr

The seed of *Brassica rapa* Jietou No.2 was treated by 0.5% EMS. By mutagenesis with EMS, a *Brassica rapa* mutant *bgr*-*JT* with dwarfed plant and compact branching was obtained, which has the characteristics of ideal plant architecture. The F2 population was constructed by crossing the mutant with Beijingkuaicai. With the genome of Chinese cabbage *B. rapa* Chiifu_V3.0 (http://brassicadb.cn) as a reference genome, the *bgr* gene was initially mapped to the interval A4:14035360 bp-20192770 bp by using Mutmap technology. Fine mapping was also performed by SSR markers, narrowing the interval to A4:16312022-16495148. Only the coding region of BraA04g022200 had SNP mutation in this interval, that is, there was no SNP mutation in the coding region of BraA04g022200 in high stem plants, and all dwarf stem plants had SNP mutations. According to the functional annotation, BraA04g022200 encodes glycogen synthase kinase 3β (GSK3β) with 96.79% sequence similarity to AT2G30980 (BIL1), which is a central negative regulatory element of the Brassinolides (BRs) signaling pathway and is also involved in the growth hormone (Auxin) signaling pathway. BraA04g022200 is named as *BGR* and its gene with SNP mutation is named as *bgr.*

### 2. Cloning of BrBGR and Brbgr of Brassica rapa

In order to obtain the full-length transcripts of the genome, the total RNA from five tissues of the mutant were extracted, including root, stem, leaf, flower and silique, by using Trizol method, and after mixing them in equal quantities, the full-length transcripts were obtained by using the PacBio sequencing technology. According to the full-length transcripts, primers BrBGR-F and BrBGR-R were designed, and the full-length DNA and full-length CDS of BrBGR were obtained by using genomic DNA and cDNA respectively.

The sequence of primer BrBGR-F: agcaggctttgactttATGACCTCACTATCATTGGGGC (lowercase letters denote a linker on the vector)
Sequence of primer BrBGR-R: tgggtctagagactttccATGACCAGGCTGTGATGGGAA (lowercase letters denote a linker on the vector).

DNA extraction: genomic DNA was extracted by CTAB method. 100 mg of fresh tissue was taken and ground using liquid nitrogen, then 1 mL of CTAB extraction buffer (4.1 g NaCl dissolved in 80 mL of H₂O, 10 g of CTAB was added slowly, and water was added to 100 mL) was added; 200 µL of chloroform was added, the resultant was left for 10 min in a water bath at 65°C, and centrifuged at 10,000 rpm for 10 minutes, and the supernatant was removed; 1/2 volume of isopropanol was added, the resultant was centrifuged at 10,000 rpm for 5 minutes, and the supernatant was discarded; 500 µL of 70% ethanol was added, the resultant was centrifuged for 5 minutes, and the supernatant was discarded; after drying, TE buffer (10mM Tris-HCl, 1mM EDTA, pH8.0) was added for later use.

Extraction of total RNA: the sample was ground in liquid nitrogen, and the resulting powder was dispensed into 1.5 mL centrifugal tubes (about 0.1 g sample per tube); 1 mL Trizol was added into the centrifuge tubes, the resultant was subjected to vortex and mixed well, and the resultant was left at room temperature for 10 minutes; 200 µL of chloroform was added, the resultant was subjected to vortex and mixed well, and the resultant was left at room temperature for 5 minutes; the resultant was centrifuged at 4°C and 13,000 rpm for 15 minutes, and the supernatant was taken into a new centrifuge tube; 500 µL of isopropanol was added, the resultant was gently inverted and mixed well, and the resultant was left at room temperature for 10~15 minutes; the resultant was centrifuged at 4°C and 13,000 rpm for 15 minutes; the supernatant was discarded, 1 mL of 70% ethanol (for RNA) was added to wash the precipitate, and the resultant was centrifuged at 13,000 rpm for 5 minutes; the precipitate was blown on an ultra-clean bench for 5 to 10 minutes, and a certain amount of RNAase-free water was added to dissolve it for later use.

cDNA synthesis: an appropriate amount of total RNA without DNA was taken, and the RNA was transcribed into cDNA by using a reverse transcription kit for later use.

PCR program: 98°C for 3 min; 98°C for 10 s, 60°C for 0.5-2 min, 72°C for 2 min, and the above programs were performed for 28 to 35 cycles, then 72°C for 3 min.

PCR system: 1 µL template, 10 µL Q5^{®} High-Fidelity 2×Master Mix, 0.5 µL each for upstream and downstream primers, and 8 µL ddH₂O.

DNA or cDNA was used as a template, the fragment with the expected size was obtained by PCR amplification, and the fragment with the expected size was ligated into the entry vector pGWC (donated by Professor Chen Qijun of China Agricultural University) by in-fusion cloning. Then sequencing analysis was performed.

Enzymatic digestion of vector: 42 µL pGWC (200 ng/µL), 3 µL *Ahd*I*,* and 5 µL 10×CutSmart buffer. 37°C 2 hr, and 65°C 10 min. For later use.

Seamless cloning: 4 µL 2×in-fusion mix, 2 µL digested plasmid, and 2 µL PCR product.

Transformation into *E. coli:* the ligation product was transformed into *E. coli* DH5α by heat shock, the clones were selected, and detected by PCR and sequenced.

Sequence analysis of gene: the full-length DNA sequences of *Brbgr* and *BrBGR,* named as *gBrbgr* (SEQ ID NO:1) and *gBrBGR* (SEQ ID NO:2) respectively, whose coding region DNA sequences were both 2873 bp in length; the CDS sequences were named *Brbgr* (SEQ ID NO:3) and *BrBGR*(SEQ ID NO:5) respectively, both of which were 1233 bp in length, encode a peptide chain comprising 410 amino acids, and the amino acid sequences encoded by *Brbgr* and *BrBGR* are represented by SEQ ID NO:4 and SEQ ID NO:6, respectively.

The DNA sequence of BrBGR consists of 12 exons and 11 introns: 1-105 bp for the first exon, 106-680 bp for the first intron, 681-773 bp for the second exon, 774-962 bp for the second intron, 963-1022 bp for the third exon and 1023-1112 bp for the third intron, 1113-1385 bp for the fourth exon, 1386-1473bp for the fourth intron, 1474-1548 bp for the fifth exon, 1549-1625 bp for the fifth intron, 1626-1683 bp for the sixth exon, 1684-1781 bp for the sixth intron, 1782-1920 bp for the seventh exon, 1921-2009 bp for the seventh intron, 2010-2060 bp is the eighth exon, 2061-2169 bp for the eighth intron, 2170-2265 bp for the ninth exon, 2266-2354 bp for the ninth intron, 2355-2438 bp for the tenth exon, 2439-2555 bp for the tenth intron, 2556-2657 bp for the eleventh exon, 2658-2777 bp for the eleventh intron, and 2778-2873 bp for the twelfth exon. In contrast to *gBrBGR, gBrbgr* has a SNP mutation G2191A in the ninth exon, resulting in a change in the amino acid of the protein it encodes, that is, the amino acid at this position changes from glutamic acid (E) to lysine (K), which is labeled as E293K.

### Example 2: Cloning of bgr gene promoter in Brassica rapa

The DNA of the *bgr* gene-containing *Brassica rapa* Jietou No.2 mutant was used as a template, the promoter region of -1234 bp upstream of *bgr*/*BGR* start codon was cloned into pHZM137 binary vector by using a primer pair (BrBGR_P-F1 and BrBGR_P-R1) to replace the CaMV35S promoter therein to obtain the vector pHZM137-BrBGRpro (Figure 1).
BrBGR_P-F1: cacacttattacaggcctATGTTCGAAATCTACAAATAAGGAGCC (lowercase letters denote a linker on the vector)
BrBGR_P-R1: aacttgtgatctcgagGTGCTATTCTTCTCTCTCTCTCTCTAACTT (lowercase letters denote a linker on the vector)

The promoter sequence of the *bgr* gene in *Brassica rapa* is represented by SEQ ID NO:7.

The pHZM137 binary vector was constructed as follows: the insulator gypsy (kindly donated by Prof. Junhui Wang of Zhejiang University, see She W, Lin W, Zhu Y, Chen Y, Jin W, Yang Y, Han N, Bian H, Zhu M, Wang J. The gypsy insulator of Drosophila melanogaster, together with its binding protein suppressor of Hairy-wing, facilitates high and precise expression of transgenes in Arabidopsis thaliana. Genetics. 2010 Aug;185(4):1141-50. doi: 10.1534/genetics.110.117960. Epub 2010 Jun 1. PMID: 20516496; PMCID: PMC2922898.) was inserted at 2 sites in the vector pEarleyGate 100 (the site between *Pme*I and *Ahd*I and site *Stu*I); then a hygromycin expression cassette derived from vector pH7GWIWG2(II) was inserted at site *Pme*I*,* and finally the synthesized 3×flag (5'-GACTACAAGGATGACGATGACAAGGGCGCCGATTATAAAGATGACGATGACAAG CAATTGGACTATAAGGACGATGACGATAAATCTAGA-3') was inserted between *Avr*II and *SpeI.*

### Example 3: Effects of Brbgr and BrBGR on the plant architecture of oilseed rape

*BrBGR* and *Brbgr* were constructed into vector pHZM137-BrBGRpro by LR recombination reaction. The vector was transformed into *Agrobacterium tumefaciens* (GV3101) by heat shock method, and the resultant *Agrobacterium tumefaciens* was transformed into *Brassica napus* (Westar) by hypocotyl infection method to observe the effect of *BrBGR* and *Brbgr* expression on the plant architecture of *Brassica napus,* and the transformation method was referred to the method of Deblock et al.

Phenotypic observation of transgenic *Brassica napus*: using *BrBGR's* own promoter to drive *Brbgr* will significantly shorten the plants, shrink the leaves and reduce the branching angle of *Brassica napus*; however, there is no significant difference in appearance traits between the BrBGR-transformed lines and the wild type (Figure 2).

It can be seen that *Brbgr* plays an important role in improving plant height and branching angle.

### Example 4: Plant architecture regulation of Brassica by bgr gene in Brassica rapa

AA subgenomes were contained in both *Brassica napus* (AACC, 2n=38) and *Brassica juncea* (AABB, 2n=36). *bgr,* a dwarf mutant of *Brassica rapa* Jietou No.2, was used as the male parent, and *Brassica napus* Zheyou 50 and *Brassica juncea* FY02 (materials preserved by the State Key Laboratory of Plant Cell and Chromosome Engineering, Institute of Genetics and Development Biology, Chinese Academy of Sciences) were used as the female parents, F 1 lines with dwarfed plant architectures and compact branches can be obtained respectively by intrageneric and interspecific hybridization (Figure 3). It shows that *bgr* derived from AA genome is functionally conservative.

### Example 5: Acquisition of new germplasm of Brassica napus with ideal plant architecture characteristics

*Brassica napus* C76 was used as the female parent, and Jietou No.2 dwarf mutant *bgr-JT* was used as the male parent, intragenus and interspecific hybridization were carried out to obtain F 1 generation seeds. After F 1, the progeny with compact plant architecture and dwarfed plant height were selected to backcross with *Brassica napus* C76 (materials preserved by the State Key Laboratory of Plant Cell and Chromosome Engineering, Institute of Genetics and Development Biology, Chinese Academy of Sciences), and the progeny with compact plant architecture and dwarfed plant height were selected from the progeny of F1BC1 to backcross with *Brassica napus* C76. Buds produced by plants with compact plant architecture and dwarfed plant height in the progeny of F1BC2 were selected for microspore culture, and a dwarf and compact plant architecture DH line material, which is, *Bnbgr,* such as DHD1 (Figure 4) was obtained by haploid technology, and a DH line of *Brassica napus* with high stem, such as DH56 (Figure 4), was also obtained.

The procedure for microspore culture was as follows: the flower buds of 3 mm in size were taken, soaked in 75% ethanol for 30-60sec, soaked in 8% sodium hypochlorite for 15 min, and washed 3-5 times in sterilized pure water; the flower buds were ground and then filtered in a mortar; the resultant was washed with NLN liquid medium (0.025 mg/L cobalt chloride hexahydrate, 0.025 mg/L anhydrous copper sulfate, 36.7 mg/L ferric sodium EDTA, 10 mg/L boric acid, 18.95 mg/L manganese sulfate, 0.25 mg/L sodium molybdate, 10 mg/L zinc sulfate, 500 mg/L calcium nitrate, 125 mg/L potassium nitrate, 125 mg/L dipotassium hydrogen phosphate, 61 mg/L magnesium sulfate, 0.05 mg/L biotin, 0.5 mg/L folic acid, 800 mg/L glutamine, 30 mg/L reduced L- glutathione, 2 mg/L glycine, 100 mg/L inositol, 5 mg/L nicotinic acid, 0.5 mg/L pyridoxine hydrochloride, 100 mg/L L-serine, and 0.5 mg/L thiamine hydrochloride), centrifuged at 4°C, 2,000 rpm, and the above procedures were repeated three times; finally the resultant was suspended with NLN liquid culture medium until the microspore concentration is controlled at 10⁴-10⁵/ml; and dispensed into a 9-cm-diameter glass petri dishes, colchicine (5 mg/L) was added, the resultant was placed in an incubator at 32°C for hot shock treatment, treated in the dark for 2 days, then placed at 25°C and stood in the dark for 10-15 days, and incubated on a shaker at 25°C, 50 rpm for 10-15 days, and transferred to B5 solid medium for seedling emergence.

### Example 6: Agronomic characters of Brassica napus bgr

After many years of observation of planting in Changping Farm, Beijing, *Bnbgr* has stable plant architecture, shrunken leaves, plant height of about 50 cm, 5-6 main branches, branching angle of about 15° (Figure 5), short siliques with 5-25 grains per silique.

Compared with the control, C76, the plant height, branching angle, branch number, biomass per plant and yield per plant of *Brassica napus* mutant *bgr* were decreased by 63.57%-64.15%, 46.66%-63.98%, 11.36%-53.64%, 51.22%-56.17% and 39.88%-62.08%, respectively. However, the harvest index, silique density, silique number in main inflorescence and total silique number increased by 23.22%-33.22%, 251.04%-395.46%, 90.07%-100.94% and 37.25%-74.13%, respectively.

### Example 7: Genetic characteristics of Brassica napus Bnbgr

Reciprocal crosses between the dwarf *Brassica napus Bnbgr* line DHD1 and high-stem material DH56 (plant height of about 170 cm) showed that their progeny was compact in plant architecture, dwarfed in plant height and consistent in performance. Therefore, it can be determined that this trait is not maternal inheritance. The statistical results of trait segregation in F2 population showed that the segregation ratio of dwarf plants : intermediate plants : tall plants was 98 : 238 : 86. The Chi-square test showed that it conformed to Mendel's single gene inheritance law of 1 : 2 : 1, which confirmed that the phenotype was controlled by a single gene. It can be proved that *bgr* is an incompletely dominant (semi-dominant) inheritance controlled by a single gene.

### Example 8: Hormone changes in vivo in Brassica napus line Bnbgr

In order to analyze the changes of hormones *in vivo* in mutants, the contents of Brassinolide (BL), Castasterone (CS), 6-deoCS (6-deoxocastasterone), typhasterol (TY) and Teasterone (TE) in the seedling stage and apical meristem of dwarf *Bnbgr* line DHD1 and high-stem line DH56 were studied. Firstly, 3-5 day old seedlings or apical meristem were taken, and subjected to quickly freeze in liquid nitrogen, and grind, and the resultant was left at -80°C for later use. For the determination method, refer to the method of Xin.

The results showed that, compared with those in *Brassica napus* DH56, the contents of BL, CS, TY and 6-deoCS in both the seedling stage and apical meristem were significantly higher, except for TE, which was not detectable (Figure 6).

At the same time, Salicylic acid (SA), gibberellin A1 (GA1), gibberellin A3 (GA3), gibberellin A4(GA4), gibberellin A7 (GA7), 3-indoleacetic acid (IAA), Abscisic acid (ABA), Jasmonic acid (JA), Jasmonic Acid-Isoleucine (JA-Ile), aminocyclopropane-l-carboxylic acid (ACC), Trans-Zeatin, tZ) and Trans-Zeatin-riboside (tZR) in the apical meristem of dwarf *Bnbgr* line DHD1 and high-stem line DH56 were determined.

The results showed that compared with DH56, the contents of ACC, ABA and GA7 in DHD1 were significantly higher; and the contents of IAA, GA4, SA, JA and JA-Ile decreased significantly; however, the contents of tZ, tZR and GA3 (data not shown) were not changed significantly (Figure 7).

### Example 9: De novo assembly of the genome of a new germplasm of Brassica napus-Bnbgr

Dwarf and compact branching *Brassica napus Bnbgr* line DHD1 is a breeding material obtained by distant hybridization combined with microspore culture, and its gene arrangement and chromosome structure may be quite different from those of existing *Brassica napus.* Using CCS (Circular Consensus Sequencing) sequencing model provided by PacBio Company and HiC technology, its genome was assembled *de novo.* The assembled genome size was 1,063,158,305 bp in total, contig N50 was 10,122,184 bp, and the total number of contigs was 2,006, and contigs accounted for 99.99% of the genome. Using BUSCO (Benchmarking Universal Single-Copy Orthologs: http://busco.ezlab.org/), 2,121 single-copy genes of eudicot plants were evaluated, and the complete proportion of *Brassica napus* genome was 99.9%, and the integrity was relatively high. This provides a high-quality reference genome for the mapping of candidate genes.

### Example 10: Cloning of BGR Gene in Brassica napus

In order to further determine whether the dwarf and compact branching mutant of *Brassica napus* originated from *Brbgr* of *Brassica rapa,* F2 genetic segregation population (DHD1 as the male parent and DH56 as the female parent) was used, and preliminary localization was carried out by the method of BSA, and fine localization by the method of SSR. Finally, BnaA04T002134 located on chromosome A04 was identified as the candidate gene *Bnbgr.* The genomic DNA sequence and cDNA sequence of *Bnbgr* and *BnBGR* were cloned by using a pair of primers (BnBGR.F and BnBGR.R) based on the genomic information of *Bnbgr* line DHD1. The methodology refers to Example 1.

Primer BnBGR.F: agcaggctttgactttATGACCTCACTATCATTGGGGC (lowercase letters denote linkers on the vector).

Primer BnBGR.R: tgggtctagagactttccATGACCAGGCTGTGATGG (lowercase letters denote linkers on the vector).

Sequence analysis showed that the full-length DNA sequence of the *Bnbgr* in dwarf, compact branching *Brassica napus* was completely consistent with the *Brbgr* in dwarf, compact branching *Brassica rapa* Jietou No.2 (SEQ ID NO: 1); the genome sequence of high-stem *Brassica napus BnBGR* (gBnBGR) was completely consistent with the full-length DNA sequence of high-stem *Brassica rapa* Jietou No.2 (SEQ ID NO:2), and compared with the genome sequence of high-stem *Brassica napus BnBGR,* the full-length DNA sequence of dwarf, compact branching *Brassica napus Bnbgr* have a single base change at position 2191, that is, from G to A.

There is only one site difference between the CDS sequence of *Bnbgr* of dwarf, compact branching *Brassica napus* DHD1 and that of *BnBGR* of high-stem *Brassica napus* DH56, that is, G at position 877 is changed to A.

The cDNA sequence of *Bnbgr* of *Brassica napus* DHD1 with compact branches is completely consistent with *Brbgr* of the dwarf, compact branching *Brassica rapa* Jietou No.2 (SEQ ID NO:3); the cDNA sequence of *BnBGR* of the high-stem *Brassica napus* DH56 is completely consistent with that of *BrBGR* of *Brassica rapa* Jietou No.2 (sequence *BrBGR,*

SEQ ID NO: 5).

### Example 11: Functional Analysis of bgr

The *Brassica napus Bnbgr* line DHD1 was used as the recipient material, and CRISPR/Cas9 gene editing technology was utilized to evaluate the effect of the *bgr* gene on oilseed rape plant architecture. The specific steps are as follows:

### I. Construction of bgr gene editing vector

1. The DNA sequences of *bgr* and *BGR* were used as templates, and two specific sgRNAs for targeting exon 3 (965-984bp) and exon 4 (1133-1152bp) were designed by CRISPR v4.99 (http://crispor.tefor.net), respectively:
   K1: ATTAGTTACATGGCGGAAC
   K2: GAATCTGTAGCCATTAAGA
2. The primers of sgRNA K1 and K2 are as follows:
   K1.F: attgATTAGTTACATGGCGGAAC
   K1.R: aaacGTTCCGCCATGTAACTAAT
   K2.F: attgGAATCTGTAGCCATTAAGA
   K2.R: aaacTCTTAATGGCTACAGATTC
   Wherein, lowercase letters denote sequences on the vector.
3. Vector assembly

Synthesized K1 or K2, respectively, were diluted to 10 µM; K1.F and K1.R or K2.F and K2.R were mixed in equal amounts at 98°C, for 5 min, and then lowered by 2°C per minute to 37°C. The vector pHZM184B (Figure 8A) was digested with Bsal: 8 µL of pHZM184B (1.6 µg in total), 1 µL of *Bsa*I*,* 1 µL of CutSmart buffer, digested at 37°C for 1 hr, and inactivated at 65°C for 10 min. This was followed by ligation with T4 ligase: 4 µL of digested pHZM184B, 4 µL of the K1 or K2 annealing products, 1 µL 10× NEB T4 Buffer, 1 µL T4 ligase, and reacted at 16°C for 2 hr. Then the resultant was transformed into *E. coli* DH5α, identified by PCR, and the correct sequences identified by sequencing were preserved, and named gK1 and gK2, respectively (Figure 8B and Figure 8C).

The pHZM184B vector was constructed as follows: Cas9 (kindly donated by Prof. Qi Xie, Institute of Genetics and Development Biology, Chinese Academy of Sciences) sequence was inserted into *XhoI* and *PacI* of vector pHZM 13 7; and then sgRNA originating from vector pHSE401 (kindly donated by Prof. Qijun Chen, College of Biological Sciences, China Agricultural University) was expressed and inserted between *EcoR*1 and *StuI.*

The literature for pHES401 (Xing HL, Dong L, Wang ZP, Zhang HY, Han CY, Liu B, Wang XC, Chen QJ. A CRISPR/Cas9 toolkit for multiplex genome editing in plants. BMC Plant Biol. 2014 Nov 29;14:327. doi: 10.1186/s12870-014-0327-y. PMID: 25432517; PMCID: PMC4262988.).

### II. Genetic transformation of oilseed rape

1. Transformation of *Agrobacterium tumefaciens* GV3101 was performed by heat shock method, identified by PCR, and monoclonal clones containing target plasmids were obtained.
2. Genetic transformation of oilseed rape. The typocotyl infestation method was utilized to transform dwarf, compact branching *Brassica napus Bnbgr* line DHD1 and wild-type *Brassica napus* Westar. 14 and 15 transgenic lines were obtained for K1 and K2 editing sites in the *Bnbgr* background, respectively; 2 and 5 transgenic lines were obtained in the Westar background, respectively.

### III. Analysis of knockout mutation types and phenotypes

Primers were designed to amplify 250-300 bp DNA fragments centered on the editing sites K1 or K2, using genomic DNA as a template. The primers were as follows (5'-3'):
K1-F: GAACCTAAACAGGTTTGAGTTCC
K1-R: GATAACAGCGAGTGAAAAGCAC
K2-F: CACTCGCTGTTATCATTTGTAG
K2-R: GAGCTCATCTCTACTCGTAGTAG

The DNA of the transgenic material was extracted and amplified by PCR to obtain the target fragment, then recovered by PCR to create a library, and the editing type of the editing target was determined by next-generation sequencing paired-ended sequencing.

The assay results showed that in DHD1, at the K1 and K2 sites, knockout mutants of *bgr* were obtained. Phenotypically, all *bgr* deletion mutants showed restoration of plant height, greater branching angle, and no longer wrinkled leaves (Figure 9).

In the Westar background, both at K1 and K2 sites, knockout mutants were obtained, but all *BGR* deletion editing mutants had no visible phenotype.

The above results further confirmed the ability of *bgr* to function in dwarfing, compact branching, and leaf crumpling plants.

### Example 12: Homologous genes of Bnbgr in Brassica napus all have the function of regulating oilseed rape plant architecture

The genome sequences of oilseed rape, *Arabidopsis thaliana,* rice, maize, soybean, grape and *Chlamydomonas reinhardtii* were downloaded from Ensembl Plants (http://plants.ensembl.org/index.html), and combined with the assembled genome of the *Brassica napus* mutant line DHD 1, the homologous genes of *Bnbgr* (BnaA04T002134) that is located on chromosome A04 of *Brassica napus* mutant DHD1, were obtained by Orthofinder analysis, there are seven homologous genes in total, with one copy each on chromosomes A01 (BnaA01T000997), A03 (BnaA03T004751), A08 (BnaA08T002216), C03 (BnaC03T001151), and C07 (BnaC07T004394), and 2 copies on chromosome C04 (BnaC04T001741 and BnaC04T005325). In order to analyze whether the mutation at the *bgr* specific site in different copies is functionally conserved in the regulation of oilseed rape plant architecture, the functions of mutants with site-specific mutation of said remaining seven copies were comparatively analyzed.

### 1. Cloning of different BGR/bgr copy CDSs from dwarf Brassica napus DHD1

The sequences of different copies of the whole genome sequence were used as a template, primers were designed as shown below, the cDNA was used as a template for PCR and the BGR/bgr copy CDS was cloned into the entry vector pGWC. Refer to Example 1 for experimental methods.
BnaA01T000997.F: agcaggctttgactttATGGCTGACGATAGGGAGATGCCG (lowercase letters denote overlapping sequence on the vector)
BnaA01T000997.R: tgggtctagagactttccAG TTCCAGACTGATTCAAGAAGCT (lowercase letters denote overlapping sequence on the vector)
BnaA03T004751 F: agcaggctttgactttATGGCTGACGATAAGGAGATGCCG (lowercase letters denote overlapping sequence on the vector)
BnaA03T004751.R: tgggtctagagactttccAGTTCCAGACTGATTCAAGAAACT (lowercase letters denote overlapping sequence on the vector)
BnaA08T002216.F: agcaggctttgactttATGGCTGACGATAAGGAGGTGCCG (lowercase letters denote overlapping sequence on the vector)
BnaA08T002216.R: tgggtctagagactttccAGTTCCAGACTGATTCAAGAAACT (lowercase letters denote overlapping sequence on the vector)
BnaC03T001151.F: agcaggctttgactttATGGCTGACGATAAGGAGGTGCCT (lowercase letters denote overlapping sequence on the vector)
BnaC03T001151 .R: tgggtctagagactttccAGTTCCAGACTGATTCAAGAAACT (lowercase letters denote overlapping sequence on the vector)
BnaC07T004394.F: agcaggctttgactttATGGCTGCCGATAAGGAGATGCCA (lowercase letters denote overlapping sequence on the vector)
BnaC07T004394.R: tgggtctagagactttccAGTTCCAGACTGATTCAAGAAACT (lowercase letters denote overlapping sequence on the vector)
BnaC04T001741.F: agcaggctttgactttATGACATCCTCCATACCATTGGGT (lowercase letters denote overlapping sequence on the vector)
BnaC04T001741.R: tgggtctagagactttccATGACCAGCTTGTAATGGAAAGCC (lowercase letters denote overlapping sequence on the vector)
BnaC04T005325 .F: agcaggctttgactttATGACATCACTATCATTGGGCCCT (lowercase letters denote overlapping sequence on the vector)
BnaC04T005325.R: tgggtctagagactttccATGACCAGGCTGTGATGGGAAGCC (lowercase letters denote overlapping sequence on the vector)

The CDS sequence of *BnaA01 T000997* is as represented by SEQ ID NO:8, and the amino acid sequence encoded by *BnaA01T000997* is as represented by SEQ ID NO:9. The CDS sequence of *BnaA03T004751* is as represented by SEQ ID NO:10, and the amino acid sequence encoded by *BnaA03T004751* is as represented by SEQ ID NO:11. The CDS sequence of *BnaA08T002216* is as represented by SEQ ID NO:12, and the amino acid sequence encoded by *BnaA08T002216* is as represented by SEQ ID NO:13. The CDS sequence of *BnaC03T001151* is as represented by SEQ ID NO: 14, and the amino acid sequence encoded by *BnaC03T001151* is as represented by SEQ ID NO: 15.The CDS sequence of *BnaC07T004394* is as represented by SEQ ID NO: 16, and the amino acid sequence encoded by *BnaC07T004394* is as represented by SEQ ID NO:17.The CDS sequence of *BnaC04T001741* is as represented by SEQ ID NO: 18 and the amino acid sequence encoded by *BnaC04T001741* is as represented by SEQ ID NO:19. The CDS sequence of *BnaC044T005325* is as represented by SEQ ID NO:20, and the amino acid sequence encoded by *BnaC044T005325* is as represented by SEQ ID NO:21.

### 2. Site-directed mutagenesis

Based on the comparative analysis of protein sequences from different species sources (Figure 10), it is found that the BGR mutation site is located in the conserved motif LGTPTRE, which is conserved in both animals and plants.

The site-directed mutagenesis technique was used to mutate nucleotide G to nucleotide A at positions 787, 787, 787, 787, 880, 877, and 787 in the CDS sequences of BnaA01 T000997, BnaA03T004751, BnaA08T002216, BnaC03T001151, BnaC04T001741, BnaC04T005325 and BnaC07T004394, respectively. The encoded corresponding amino acid was mutated from E to K, which resulted in CDS mutants of different copies, named mBnaA01T000997, mBnaA03T004751, mBnaA08T002216, mBnaC03T001151, mBnaC04T001741, mBnaC04T005325 and mBnaC07T004394, respectively.

The primers for site-directed mutagenesis of different copies of CDS in *Brassica napus* were as follows:
The primers for site-directed mutagenesis of BnaA01T000997, BnaA03T004751, BnaA08T002216, BnaC03T001151, and BnaC07T004394 are as follows, with the same orientation (5'-3'):
m01.F: ACCAACTCGAaAAGAGATCCG
m01.R: CGGATCTCTTtTCGAGTTGGT

The primers for site-directed mutagenesis of BnaC04T001741 and BnaC04T005325 are as follows, respectively, with the same orientation (5'-3'):
mBnaC04T001741.F: TCCAACTCGAaAAGAGATCAG
mBnaC04T001741.R: CTGATCTCTTtTCGAGTTGGA
mBnaC04T005325.F: TCCAACTCGAaAAGAAATCCG
mBnaC04T005325.R: CGGATTTCTTtTCGAGTTGGA

Wherein, lowercase letters denote mutated bases.

PCR system for site-directed mutagenesis: 1 µL of plasmids (100 ng/µL) containing the target gene, 10 µL of Q5^{®} High-Fidelity 2×Master Mix, 0.5 µL each of upstream and downstream primers, and 8 µL of ddH₂O.

PCR program: 98°C for 3 min; 25 cycles (98°C for 10 sec, 60°C for 10 sec, 72°C for 2 min); 72°C for 5 min.

PCR product digestion: 1 µL *Dpn*I*,* 1 µL CutSmart, 8µL PCR product, incubated at 37°C for 2 hr.

Detection of gene mutation: the digested PCR product was transformed into *E. coli* DH5α, clones were randomly picked, sequenced and identified, and clones with site-directed mutations were selected.

The CDS sequence of the mutated *BnaA01T000997, mBnaA01T000997,* is represented by SEQ ID NO:22, and the amino acid sequence encoded by *mBnaA01T000997* is represented by SEQ ID NO:23. The CDS sequence of the mutated *BnaA03T004751, mBnaA03T004751,* is represented by SEQ ID NO:24 is shown, and the amino acid sequence encoded by *mBnaA03T004751* is represented by SEQ ID NO:25. The CDS sequence of the mutated *BnaA08T002216, mBnaA08T002216,* is represented by SEQ ID NO:26, and the amino acid sequence encoded by *mBnaA08T002216* is represented by SEQ ID NO:27. The CDS sequence of the mutated *BnaC03T001151, mBnaC03T001151,* is represented by SEQ ID NO:28 and the amino acid sequence encoded by *mBnaC03T001151* is represented by SEQ ID NO:29. The CDS sequence of the mutated *BnaC04T001741, mBnaC04T001741,* is represented by SEQ ID NO:30 and the amino acid sequence encoded by *mBnaC04T001741* is represented by SEQ ID NO:31. The CDS sequence of the mutated *BnaC04T005325, mBnaC04T005325,* is represented by SEQ ID NO:32 and the amino acid sequence encoded by *mBnaC04T005325* is represented by SEQ ID NO:33. The CDS sequence of the mutated *BnaC07T004394, mBnaC07T004394,* is represented by SEQ ID NO:34 and the amino acid sequence encoded by *mBnaC07T004394* is represented by SEQ ID NO:35.

### 3. Functional analysis of different copy mutants of Brassica napus

The mutants of different copies were recombined into pHZM137-BrBGRpro by LR recombination. After the transformation into Agrobacterium, genetic transformation into oilseed rape (Westar) was performed. Transgenic lines were obtained and analyzed phenotypically.

Phenotypically, all transgenic materials with different gene mutants were phenotypically similar to the *bgr* transgenic plants, i.e., dwarfed plants with compact branching and crumpled leaves (Fig. 11). This indicates that the proteins encoded by these homologous genes in oilseed rape have their conserved motifs LGTPTRE with E mutated to K. The mutated proteins are conserved for the regulation of plant phenotypes of *Brassica napus,* and all of them resulted in dwarfed and compact *Brassica napus* plants. For Different copies (BnaA01T000997, BnaA03T004751, BnaA08T002216, BnaC03T001151, BnaC04T001741, BnaC04T005325, and BnaC07T004394) the mutation from E in LGTPTRE motif to K can realize the regulation of plant architecture of *Brassica napus.*

### Example 13: Site-directed mutagenesis was achieved by prime editing

### 1. Artificial synthesis and vector construction for prime editing

*BnBGR* (5'-GAgAAGAAATCCGATGCATG-3', g as the target mutation site) was used as the target, the prime editing sequence was designed and synthesized according to Jiang et al., and it was constructed into the vector pZ1WS (kindly donated by Professor Chen Qijun of China Agricultural University) to obtain pZ1WS-PEbgr (Figure 12). Refer to Example 11 for the construction method.

The prime editing sequence is as follows (5'-3') (SEQ ID NO:36):

CATGCATCGGATTTCTTCTCGTTTTAGAGCTAGAAATAGCAAGTTAAAATA
AGGCTAGTCCGTTATCAACTTGAAAAAGTGGCACCGAGTCGGTGCCCAACCCGA
AAAGAAATCCGATgccggcatggtcccagcctcctcgctggcgccggctgggcaacatgcttcggcatggcgaatggg
act

Wherein, the portion of the sequence in bold denotes gRNA; the portion marked with wave line denotes scaffold RNA; the portion marked with single underline denotes template for reverse transcription; the portion marked with double underline, PBS (primer binding site); lowercase letters, HDV (ribozyme from hepatitis delta virus).

### 2. Genetic transformation of oilseed rape

Genetic transformation was carried out with Westar as the recipient material, and the method was referred to Example 3.

### 3. Phenotypic analysis

The progeny of Westar after gene editing showed the phenotypes of dwarfing, compact plant architecture and smaller branch angle, which was similar to the phenotype of DHD1 and *bgr* transgenic - plants described previously. By analyzing these gene editing materials, it was found that the editing target site of Westar has mutated from G to A. It can be seen that the mutant of this site can be obtained by prime editing.

### Example 14: Brassica napus Bnbgr has significant heterosis

### 1. Breeding evaluation of Brassica rapa diploid mutant bgr-JT

The F1 seeds obtained by crossing the homozygous mutant of *Brassica rapa* with Yellow Sarson were used to evaluate the potential of *bgr-JT* in diploid hybrid breeding. The results showed that the homozygous mutant *bgr-JT* was extremely dwarfed and had poor fertility. The plant height of F1 is between wild type and mutant, and it was compact branching. According to the results of the single-plant examination, compared with the wild type, the plant height, silique length, silique number and yield per plant of F1 decreased by about 30%, and the yield of a single plant was reduced by about 50%; the density of silique was significantly increased by about 60%.

It can be seen that the gene has no potential to increase the yield of the *Brassica rapa* materials under a diploid background.

### 2. Evaluation on breeding potential of tetraploid Brassica napus mutant

The hybrid F1 was obtained by crossing the obtained *Brassica napus bgr* with *Brassica napus* (C76), and the hybrid F1 had significant heterosis.

Analysis of individual plant characters: the plant height of hybrid F1 is 120.57±8.56 cm, and the branching angle is less than 30°. The data showed that compared with the male parent, the plant height, the height of initial branch position in the stem and internode spacing of F1 decreased significantly, by 30.10±2.91%, 64.29±13.64% and 43.76±13.17% respectively. The number of branches (primary effective branch), the number of siliques per plant and the yield per plant were significantly higher than those of their parents, increasing by 42.86±13.67%, 49.09±9.18% and 45.34±6.44% respectively. There was no significant difference in biomass compared with the male parent, but the harvest index was significantly higher than that of the male parent, with an increase of 30.77±3.18%.

It can be known that this gene has breeding potential in tetraploid background. For this reason, yield analysis of F1 hybrid of *Brassica napus* was also carried out: *Brassica napus bgr* can be used as the maintainer line for the cytoplasmic male sterile system of *Brassica napus,* and the CMS sterile line 50A (female parent, materials preserved by the State Key Laboratory of Plant Cell and Chromosome Engineering, Institute of Genetics and Development Biology, Chinese Academy of Sciences) was crossed with *bgr* plant architecture (male parent), and then the *bgr* was backcrossed for 5 generations to obtain the sterile line *bgrA.*

*BnbgrA* was hybridized with a restorer line of *Brassica napus* (PM112) to obtain F1 hybrid. In October 2021, the productivity of hybrid was measured in Luoping, Yunnan and Yangling, Shaanxi, respectively. It was harvested in May 2022, and the test results are as follows:
The planting area in Yangling, Shaanxi Province is 74.4 m², the planting density is about 35,000 plants/mu (i.e., 525,000 plants/ha), the plant height is about 130 cm at harvest, with lodging resistance, the equivalent mu yield can reach 282.27 kg (i.e., 4234.05 kg/ha), and the harvest index is about 0.40. The yield of the control Shanyou 1203 per mu is 252.98 kg (3794.70 kg/ha), and its harvest index is about 0.31. The yield and harvest index of hybrid increased by 11.57% and 29.03% respectively. The oil content of the hybrid measured by near-infrared spectroscopy and nuclear magnetic resonance (NMR) method is up to 51.7% to 54.86%, which was 9.14% to 19.26% higher than that of the control (46.00% to 47.37%).

A large-scale production demonstration was carried out in Luoping, Yunnan Province, which was harvested in May 2022. The planting area is 656.6 m², the planting density is about 28,000 plants/mu (i.e., 420,000 plants/ha), the plant height was about 150 cm, with lodging resistance, the equivalent mu yield can reach 257.9 kg (i.e., 3868.50 kg/ha), and the yield per mu was 27.15% higher than that of the control Yunyouza 15 (200.83 kg/mu, i.e., 3012.45 kg/ha 3868.50 kg/ha).

Although the present application has been described in detail above with general descriptions and specific embodiments, it is obvious to those skilled in the art that some modifications or improvements can be made on the basis of the present application. Therefore, these modifications or improvements made without departing from the spirit of the application belong to the scope of the application.

### Industrial applicability

The present application provides an oilseed rape green revolution gene *bgr* and use thereof. The gene *bgr* can significantly reduce the height of oilseed rape plants, make the stems thick and strong, realize lodging resistance, reduce the branching angle, compact the plant architecture and effectively improve the harvest index. The gene has a significant heterosis, and the heterozygous *bgr* can enable tetraploid oilseed rape (*Brassica napus* and *Brassica juncea*) to have more ideal plant architecture characteristics, wherein the plant height is 120 to 160 cm, the plant architecture is compact, and the branching angle is small (<30°), the position of the first branching is lowered, the harvest index is improved by about 29.03%, the method is suitable for dense planting, the yield is significantly increased by 11% to 27%. The oil content of hybrid *Brassica napus* with *bgr* is substantially increased up to 51.7% to 54.86%, which is 9.14% to 19.26% higher than that of common high-oil oilseed rape varieties. The present application contributes valuable gene resources for oilseed rape breeding, provides an effective means for the creation of new oilseed rape germplasm, and has good economic value and application prospects.

## Claims

1. An oilseed rape green revolution gene *bgr,* wherein the gene *bgr* is a gene encoding the following protein (a) or (b):
(a1) a protein comprising the amino acid sequence represented by SEQ ID NO:4;
(b1) a protein derived from (a1) by substitution, deletion or addition of one or several amino acids in the sequence represented by SEQ ID NO:4 and having an equivalent function.

2. A homologous gene of the oilseed rape green revolution gene *bgr* according to claim 1, wherein there are seven homologous genes in total, which are mBnaA01T000997, mBnaA03T004751, mBnaA08T002216, mBnaC03T001151, mBnaC04T001741, mBnaC04T005325 and mBnaC07T004394, respectively;
wherein, mBnaA01T000997 is a gene encoding the following protein (a2) or (b2):
(a2) a protein comprising the amino acid sequence represented by SEQ ID NO:23;
(b2) a protein derived from (a2) by substitution, deletion or addition of one or several amino acids in the sequence represented by SEQ ID NO:23 and having an equivalent function;
mBnaA03T004751 is a gene encoding the following protein (a3) or (b3):
(a3) a protein comprising the amino acid sequence represented by SEQ ID NO:25;
(b3) a protein derived from (a3) by substitution, deletion or addition of one or several amino acids in the sequence represented by SEQ ID NO:25 and having an equivalent function;
mBnaA08T002216 is a gene encoding the following protein (a4) or (b4):
(a4) a protein comprising the amino acid sequence represented by SEQ ID NO:27;
(b4) a protein derived from (a4) by substitution, deletion or addition of one or several amino acids in the sequence represented by SEQ ID NO:27 and having an equivalent function;
mBnaC03T001151 is a gene encoding the following protein (a5) or (b5):
(a5) a protein comprising the amino acid sequence represented by SEQ ID NO:29;
(b5) a protein derived from (a5) by substitution, deletion or addition of one or several amino acids in the sequence represented by SEQ ID NO:29 and having an equivalent function;
mBnaC04T001741 is a gene encoding the following protein (a6) or (b6):
(a6) a protein comprising the amino acid sequence represented by SEQ ID NO:31;
(b6) a protein derived from (a6) by substitution, deletion or addition of one or several amino acids in the sequence represented by SEQ ID NO:31 and having an equivalent function;
mBnaC04T005325 is a gene encoding the following protein (a7) or (b7):
(a7) a protein comprising the amino acid sequence represented by SEQ ID NO:33;
(b7) a protein derived from (a7) by substitution, deletion or addition of one or several amino acids in the sequence represented by SEQ ID NO:33 and having an equivalent function;
mBnaC07T004394 is a gene encoding the following protein (a8) or (b8):
(a8) a protein comprising the amino acid sequence represented by SEQ ID NO:35;
(b8) a protein derived from (a8) by substitution, deletion or addition of one or several amino acids in the sequence represented by SEQ ID NO:35 and having an equivalent function.

3. A biomaterial containing the oilseed rape green revolution gene *bgr* of claim 1 or the homologous gene of claim 2, wherein the biomaterial is a recombinant DNA, an expression cassette, a transposon, a plasmid vector, a virus vector or an engineered bacterium.

4. Any of the following uses of the oilseed rape green revolution gene *bgr* of claim 1 or the homologous gene of claim 2 or the biomaterial of claim 3:
(1) for regulating plant height, plant architecture, leaf type, oil content, harvest index and yield;
(2) for improving plant variety;
(3) for preparing transgenic plants;
wherein the plant architecture includes branching angle and branch number; and
the plant is Brassica, preferably oilseed rape, more preferably tetraploid oilseed rape, most preferably *Brassica napus* and *Brassica juncea.*

5. A method for reducing plant height, reducing branching angle, compacting plant architecture, shrinking leaves, increasing oil content, increasing harvest index and increasing yield of oilseed rape, wherein the method comprises: introducing mutation into oilseed rape genome by means of genetic engineering, gene editing, chemical and/or physical mutagenesis and plant hybridization, so that the E in the conserved motif LGTPTRE in the encoded glycogen synthase kinase 3β and its homologous protein is mutated into K; or,
introducing the oilseed rape green revolution gene *bgr* of claim 1 or the homologous gene of claim 2 into oilseed rape via plasmid or integrating the oilseed rape green revolution gene *bgr* of claim 1 or the homologous gene of claim 2 into oilseed rape chromosome by genetic engineering;
preferably, the oilseed rape is tetraploid, more preferably *Brassica napus* and *Brassica juncea.*

6. The method according to claim 5, wherein the method comprises: overexpressing the oilseed rape green revolution gene *bgr* or the homologous gene in plants;
the overexpression mode is selected from the following modes 1) to 5), or any combination thereof:
1) by introducing a plasmid having the gene;
2) by increasing the copy number of the gene on the plant chromosome;
3) by changing the promoter sequence of the gene on the plant chromosome;
4) by operably linking a strong promoter or a weak promoter with the gene;
5) by introducing an enhancer.

7. The method according to claim 6, wherein the promoter is the promotor of *bgr* or *BGR* gene encoding GSK3β protein in *Brassica rapa,* and a nucleotide sequence of the promoter is:
i) the nucleotide sequence represented by SEQ ID NO:7;
ii) the nucleotide sequence obtained by substitution, deletion and/or addition of one or more nucleotides in the sequence represented by SEQ ID NO:7 and having an equivalent function;
iii) a nucleotide sequence that hybridizes to the sequence represented by SEQ ID NO:7 under stringent conditions, wherein the stringent conditions are hybridization in a solution of 0.1 × SSPE containing 0.1% SDS or a solution of 0.1 × SSC containing 0.1% SDS at 65°C and washing the membrane with the solution; or
iv) a nucleotide sequence having 90% or more homology with the nucleotide sequence of i), ii) or iii) and having the same promoter function.

8. Use of transgenic oilseed rape obtained by the method according to any one of claims 5 to 7 in plant breeding;
wherein the plant is *Brassica,* preferably oilseed rape, more preferably tetraploid oilseed rape, most preferably *Brassica napus* and *Brassica juncea.*

9. The use according to claim 8, wherein the breeding method comprises transgenesis, hybridization, backcrossing, self-crossing or asexual reproduction;
preferably, the obtained dwarf and compact oilseed rape is used as a maintainer line for the creation of a sterile line or a restorer line of oilseed rape, on the basis of the sterile line and the restorer line of oilseed rape, hybrids are produced.

10. A method for creating new germplasms of *Brassica napus,* wherein a mutation is introduced into the genome of diploid oilseed rape by using genetic engineering technology, gene editing technology or mutagenesis technology, so that the E in the encoded conserved motif LGTPTRE in glycogen synthase kinase 3β is mutated into K, and an oilseed rape mutant with ideal plant architecture characteristics is obtained; then the oilseed rape mutant is hybridized with *Brassica napus* in combination with microspore culture technology to obtain *Brassica napus* breeding materials;
preferably, CRISPR/Cas9 gene editing technology is used to introduce the mutation in the diploid oilseed rape genome, and the prime editing sequence is represented by SEQ ID NO:36.
